# FASCICULE DE BREVET EUROPEEN

(11) **EP 1 281 081 B1**
(45) Date de publication et mention de la délivrance du brevet: **16.01.2008**
(21) Numéro de dépôt: 01931796.5
(22) Date de dépôt: 04.05.2001
(51) Int. Cl.: G01N 33/50, C12Q 1/68, C12N 7/02

(54) **PROCEDE DE DETERMINATION D'AGENTS BIOLOGIQUES DANS DES CELLULES CIBLES VIVANTES**
VERFAHREN ZUR BESTIMMUNG VON BIOLOGISCHEN AGENZIEN IN LEBENDEN ZIELZELLEN
METHOD FOR DETERMINING BIOLOGICAL AGENTS IN LIVING TARGET CELLS

(30) Priorité: 09.05.2000 FR 0005852
(43) Date de publication de la demande: 05.02.2003
(73) Titulaire: Nautilus Biotech, 91000 Evry Cédex (FR)
(72) Inventeur: VEGA, Manuel, F-91270 Vigneux-sur-Seine (FR)
(74) Mandataire: Vialle-Presles, Marie José
(86) Numéro de dépôt international: PCT/FR2001/001366
(87) Numéro de publication internationale: WO 2001/086291

(56) Documents cités:
- WO-A-99/11764
- CHARBORD P; NEEL H; LEHN P; PARMENTIER C: "NORMAL HUMAN GRANULO MONOCYTIC BONE MARROW PROGENITOR CELLS RESPONSIVENESS TO COLONY STIMULATING ACTIVITY" NOUVELLE REVUE FRANCAISE D'HEMATOLOGIE, vol. 22, 1980, pages 357-370, XP000949859
- SCHUHMANN KLAUS; ROMANIN CHRISTOPH; BAUMGARTNER WERNER; GROSCHNER KLAUS: "Intracellular Ca2+ inhibits smooth muscle L-type Ca2+ channels by activation of protein phosphatase type 2B and by direct interaction with the channel" JOURNAL OF GENERAL PHYSIOLOGY, vol. 110, novembre 1997 (1997-11), pages 503-513, XP000949861
- MOULLIER P; DAVELOOSE D; LETERRIER F; HOEBEKE J: "COMPARATIVE BINDING OF WHEAT GERM AGGLUTININ AND ITS SUCCINYLATED FORM ON LYMPHOCYTES" EUROPEAN JOURNAL OF BIOCHEMISTRY, vol. 161, 1986, pages 197-204, XP000951462
- DAVIS A R ET AL: "HIGH THROUGHPUT METHOD FOR CREATING AND SCREENING RECOMBINANT ADENOVIRUSES" GENE THERAPY,GB,MACMILLAN PRESS LTD., BASINGSTOKE, vol. 5, no. 8, août 1998 (1998-08), pages 1148-1152, XP000867556 ISSN: 0969-7128
- ATKINSON E M ET AL: "A high-throughput hybridization method for titer determination of viruses and gene therapy vectors" NUCLEIC ACIDS RESEARCH,GB,OXFORD UNIVERSITY PRESS, SURREY, vol. 26, no. 11, 1 juin 1998 (1998-06-01), pages 2821-2823, XP002099502 ISSN: 0305-1048 cité dans la demande
- NELSON DAVID M; WAHLFORS J JARMO; CHEN LIN; ONODERA MASAFUMI; MORGAN RICHARD A: "Characterization of diverse viral vector preparations, using a simple and rapid whole-virion dot-blot method" HUMAN GENE THERAPY, vol. 9, 1 novembre 1998 (1998-11-01), pages 2401-2405, XP000951429 cité dans la demande
- MITTEREDER NANETTE; MARCH KEITH L; TRAPNELL BRUCE C: "Evaluation of the concentration and bioactivity of adenovirus vectors for gene therapy" JOURNAL OF VIROLOGY, vol. 70, no. 11, novembre 1996 (1996-11), pages 7498-7509, XP002148995 cité dans la demande
- SALVETTI A ET AL: "Factors influencing recombinant adeno-associated virus production" HUMAN GENE THERAPY,XX,XX, vol. 9, no. 5, 20 mars 1998 (1998-03-20), pages 695-706, XP000946374 ISSN: 1043-0342 cité dans la demande
- BIOLOGICAL ABSTRACTS, vol. 260, 01 Février 2002, Philadelphia, PA, US; REBELLO; HALE: 'Pharmacokinetics of CAMPATH-IH: assay development and validation' page 285;

## Description

L'invention est relative à un procédé de détermination du titre (concentration) d'agent biologiques, sélectionné dans le groupe constitué par les virus et les vecteurs viraux de transfert de gènes, en temps réel, dans des cellules cibles vivantes, ainsi qu'à ses applications (thérapie génique, génomique fonctionnelle, diagnostic viral, vaccins, protéines recombinantes).

Les progrès relatifs aux transferts de gènes en thérapie génique dépendent d'une part de la capacité à développer et à produire des vecteurs permettant, dans la cellule cible, une expression régulée d'une protéine ou d'un ARN qui possèdent des effets thérapeutiques et d'autre part de la capacité à identifier de nouveaux gènes thérapeutiques.

Ainsi, avec le développement récent du domaine de la génomique fonctionnelle, les vecteurs initialement développés pour le transfert de gènes, sont aussi utilisés comme outils pour le criblage des banques de gènes.

Ces progrès impliquent :
- la construction et le criblage de banques de vecteurs de transfert de gènes,
- le développement de constructions de vecteurs optimalisées et parfaitement adaptées à chaque application thérapeutique, notamment en terme de ciblage tissulaire et de régulation de l'expression et
- la production en grande quantité de vecteurs contrôlés, standardisés et de qualité optimale, permettant de réaliser des études précliniques et des essais cliniques de phase I.

Dans ce contexte, afin d'analyser rapidement de nombreuses constructions de vecteurs et d'optimaliser leur production en grande quantité, il est important de pouvoir déterminer facilement, rapidement et précisément la concentration desdits vecteurs par des méthodes performantes.

Les méthodes de détermination de la concentration ou du titre desdits vecteurs, en particulier des vecteurs viraux, décrites dans la littérature se divisent en méthodes physiques et en méthodes biologiques.

Les méthodes physiques mesurent le titre en particules physiques (*pp*) (Mittereder et al., J. Virol., 1996, 70, 11, 7498-7509 ; Atkinson et al., NAR, 1998, 26, 11, 2821-2823 ; Nelson et al., Hum. Gene Ther., 1998, 9, 16, 2401-2405), qui représente le nombre total de particules virales de vecteur ; habituellement ce titre est évalué soit directement par comptage des particules virales en microscopie électronique, soit indirectement par mesure du contenu en acides nucléiques (hybridation ou absorbance des acides nucléiques (DO₂₆₀) pour AAV et AdV, respectivement), ou en protéines virales (activité RT et contenu en p24, par exemple pour MLV et HIV, respectivement) des vecteurs. La mesure du titre en particules physiques ne reflète pas la quantité de particules infectieuses présentes et biologiquement actives, en raison de la présence de particules défectives *(defective-interfering particles ou DI*) non infectieuses, sans génome ou avec un génome incomplet.

Les méthodes biologiques permettent, en revanche, de déterminer un titre en particules infectieuses (*ip* : unités infectieuses, unités formant plage, unités de transduction) (Mittereder et al., précité ; Salvetti et al., Hum. Gene Ther., 1998, 9, 5, 695-706 ; Atkinson et al., NAR, 1998, 26, 11, 2821-2823) par la mesure d'un paramètre biologique qui reflète l'activité du vecteur dans des cellules infectées en culture : réplication virale (AAV), intégration du provirus (rétrovirus, HIV), lyse cellulaire (formation de foyers ou de plages de lyse, uniquement dans le cas de virus lytiques (AdV, HSV)) et expression du transgène (tous les types de vecteurs). *ip* mesure le nombre de particules actives dans le processus biologique dont l'effet est mesuré. Ainsi, les préparations de vecteur présentant un titre élevé en particules infectieuses et un rapport particules physiques/particules infectieuses faible sont considérées comme étant de haute qualité, ces deux paramètres étant considérés comme fournissant une information quantitative concernant la performance d'une préparation d'un vecteur de transfert de gène.

Quelle que soit la nature du paramètre mesuré, les méthodes décrites reposent essentiellement sur une dilution en série du vecteur (environ 10 à 20 dilutions en double ou en triple), suivie d'une période d'incubation du vecteur avec les cellules (1 à 15 jours), puis du traitement des cellules (lyse, fixation, coloration, addition de substrat, hybridation, PCR), de la mesure du paramètre fonctionnel et enfin de la détermination du titre qui correspond à la dilution limite, c'est-à-dire à la dilution la plus élevée pour laquelle la valeur du paramètre biologique mesuré atteint sa limite de détection.

Le titre est généralement déterminé à partir de la courbe qui représente les valeurs du paramètre biologique en fonction de la dilution du vecteur :
- par une extrapolation linéaire à partir de la région centrale quasi-linéaire de la courbe suivie de la détermination de l'intersection avec l'axe des abscisses ou
- par une approximation asymptotique de ladite courbe dans la région des dilutions élevées ; une telle approximation peut être effectuée à l'aide d'un programme informatique, qui repose sur une fonction hyperbolique pour le calcul du titre.

Ainsi, plus le nombre de dilutions testées est élevé, plus la valeur du titre sera précise.

Cependant ces techniques sont peu fiables et présentent l'inconvénient de ne pas être standardisées. Pour résoudre ce problème, de nouvelles méthodes mieux adaptées à la détermination du titre (ou concentration) et à la comparaison de différents virus recombinants utilisés en thérapie génique ont été proposées (E.M. Atkinson et al, précité ; Demande Internationale PCT WO 99/11764). Par exemple, dans l'article au nom d'E.M. Atkinson et al., précité et dans la Demande Internationale PCT WO 99/11764, la méthode qui est décrite met en oeuvre essentiellement une étape d'amplification du matériel génétique viral dans une lignée cellulaire hôte, des préparations de vecteur standard de titre connu obtenues par dilutions en série et un contrôle interne de titre connu. De manière plus précise, la méthode comprend dans différents puits d'une plaque de microtitration, l'infection de cellules à l'aide de dilutions en série d'une préparation virale (10 dilutions en triplicat), la réplication du génome viral dans ladite cellule hôte pendant 48 h à 72 h, la lyse chimique de ladite cellule, une hybridation de l'acide nucléique, la mesure de la quantité relative d'acide nucléique viral répliqué dans chaque puits et la détermination du titre par extrapolation linéaire de la courbe, qui représente les valeurs des mesures obtenues en fonction de la dilution du vecteur.

Ainsi, les méthodes de l'Art antérieur, même les mieux adaptées comme celles décrites par d'E.M. Atkinson et al., précité, ne répondent pas aux besoins du développement et de la production des vecteurs de transfert de gènes pour les raisons suivantes :
- elles sont très lourdes à mettre en oeuvre et comprennent de nombreuses manipulations, à chaque étape de la méthode, en raison du nombre important d'échantillons correspondant à chaque dilution de vecteur. Par conséquent, elles ne sont pas utilisables dans le contexte du développement et de la production des vecteurs de transfert de gènes, qui implique le traitement de très nombreux échantillons pour comparer différentes constructions ou conditions de production des vecteurs ou bien pour suivre la cinétique de production de ces vecteurs,
- elles ne sont pas standardisées pour la plupart,
- elles sont difficilement automatisables, étant donné le nombre et la complexité des étapes à mettre en oeuvre, et
- le résultat est obtenu à un temps fixe qui, en fonction de la nature du paramètre mesuré, est de plusieurs jours (expression d'un transgène) à plusieurs semaines (formation de plages de lyse). Ainsi, les délais nécessaires pour la mise en oeuvre de ces méthodes ne sont pas adaptés à la détermination rapide de la concentration des vecteurs de transfert de gènes vecteurs, dans le criblage des banques de vecteurs, le contrôle en cours de production ou bien pour suivre la cinétique de production de ces vecteurs.

La présente invention s'est en conséquence donnée pour but, de fournir un procédé de détermination du titre d'un agent biologique, sélectionné dans le groupe constitué par les virus et les vecteurs viraux de transfert de gènes, qui répond mieux au besoin de la pratique, en ce qu'elle permet l'analyse de nombreux échantillons en temps réel.

L'invention a également pour objet les applications dudit procédé pour le criblage, l'analyse et la production des vecteurs viraux de transfert de gènes, des vaccins viraux et des protéines recombinantes ainsi que pour le diagnostic des infections virales.

La présente invention a pour objet un procédé de détermination du titre d'un agent biologique, sélectionné dans le groupe constitué par les virus et les vecteurs viraux de transfert de gènes, interagissant avec des cellules cibles vivantes, lequel procédé est caractérisé en ce qu'il comprend au moins les étapes suivantes :
(a₁) l'incubation dudit agent biologique à une concentration C initiale inconnue, avec lesdites cellules cibles à une concentration D constante,
(b₁) la mesure à différents temps successifs t, de l'intensité *i* d'un même signal, qui résulte de la réaction agent biologique + cellules cibles vivantes,
(c₁) la détermination du temps *t*_{β} correspondant à la valeur i = β, choisie dans l'intervalle βₘᵢₙ < β < βₘₐₓ, tel que βₘᵢₙ et βₘₐₓ correspondent aux valeurs de i au point d'inflexion de la courbe *i* = f (*t*) pour respectivement, les concentrations minimales et maximales d'un agent biologique de référence, dont la courbe *t*_{β} = f (*C*) (courbe de référence), est préétablie et
(d₁) la détermination de la concentration C initiale de l'agent biologique, à l'aide de ladite courbe de référence *t*_{β} = f (*C*).

### Définitions

- on entend par agent biologique : un vecteur de transfert de gènes viral ou non-viral, un virus, un anticorps, un vaccin ou une protéine recombinante.
- on entend par cellules cibles vivantes, des cellules cibles, *in vitro* ou *ex vivo,* avant leur modification par un agent biologique.
- on entend par titre C d'un agent biologique, sa concentration en particules (virus, vecteur de transfert de gène viral, vaccin viral) ou en molécules actives (protéine recombinante, anticorps), dans la réaction agent biologique + cellules cibles vivantes (*C* correspond au titre en particules infectieuses ou *ip,* tel que défini ci-dessus pour les vecteurs viraux de transfert de gènes).
- on entend par réaction agent biologique + cellules cibles vivantes, la réponse des cellules cibles à l'agent biologique ou processus biologique, il s'agit notamment :
   - de l'expression d'un gène rapporteur ou d'un transgène,
   - de la réplication, de l'intégration ou de l'activité cytolytique d'un virus,
   - d'une activité enzymatique, anti-virale, oncogénique, suppresseur de tumeur ou cytotoxique,
   - de la prolifération ou de la différenciation cellulaire, ou
   - de la liaison à des anticorps ou à des récepteurs.
- Le produit P de la réaction agent biologique + cellules cibles vivantes est mesurable par un signal ; il est déterminé par la mesure d'un paramètre qui reflète la réponse des cellules cibles vivantes à l'agent biologique. De manière non limitative, on peut citer comme mesure : la quantité de protéine ou d'enzyme exprimée par un gène rapporteur ou un transgène, le nombre de copies de génome du vecteur viral, le nombre de cellules.
- on entend par signal, par exemple la fluorescence, la luminescence, l'absorbance ou le dénombrement de cellules. De manière non limitative, on peut citer comme technique de mesure du signal : la microscopie optique ou de fluorescence, la fluorimétrie, la luminométrie et la spectrométrie.
- on entend par mesure de l'intensité du signal, la mesure du produit P de la réaction agent biologique + cellules cibles vivantes, sans intervention sur les cellules cibles et sur ladite réaction, dont le produit P est mesuré.
- on entend par agent biologique de référence, un agent biologique identique ou similaire à l'agent biologique à analyser, qui présente des modifications qui n'affectent pas son activité dans la réaction dont le produit P est mesuré.
- on entend par mesure en temps réel, une mesure dont la valeur est obtenue instantanément.

De manière surprenante, l'Inventeur a montré que l'intensité du signal *i*, qui reflète la réponse des cellules cibles à l'agent biologique, dépend uniquement de deux paramètres : la concentration *C* et le temps t. Ainsi, lorsque *t* augmente, l'intensité du signal *i* augmente, proportionnellement à la valeur de *C*; en conséquence, pour une valeur de *C* constante, *i* varie proportionnellement à *t* et pour une valeur de t constante, *i* varie proportionnellement à *C*.

Alors que pour mesurer la concentration *C* d'un agent biologique, les méthodes de l'Art antérieur utilisent une valeur de *t* constante, l'Inventeur a trouvé, de manière inattendue que l'utilisation d'une valeur constante de C permet une détermination plus simple, plus rapide et plus précise la concentration des agents biologiques.

Ainsi, l'Inventeur a montré, de manière surprenante, que la concentration d'un échantillon biologique peut-être déterminée directement, sans avoir besoin de diluer ledit échantillon, (1) en mesurant les valeurs de *i* à différents instants t et en déterminant la valeur *t*_{β} correspondant à la valeur *i* = β puis (2) en déterminant la valeur de C correspondant à la valeur *t*_{β}, à partir de la courbe de référence t = f (C), telle que définie ci-dessus.

Selon un mode de réalisation avantageux du procédé de l'invention, la courbe de référence est établie simultanément ou préalablement à l'étape (a₁), telle que décrite ci-dessus, selon les étapes suivantes :
(a₀) la préparation, de n dilutions en série d'un agent biologique de référence de concentration *C₀* initiale connue, correspondant respectivement aux concentrations finales *C*₁, *C*₂*,* ...... *C*ₙ dudit agent biologique,
(b₀) l'incubation de chaque dilution dudit agent biologique de référence obtenue en (a₀) avec lesdites cellules cibles à une concentration *D* constante,
(c₀) la détermination à différents temps successifs *t*₁ à *t*ₙ de l'intensité du signal *i* qui résulte de la réaction agent biologique + cellules cibles vivantes, pour chaque concentration *C*₁, *C*₂,.... *C*ₙ dudit agent biologique de référence,
(d₀) le tracé de la courbe *i* = f (*t*) pour chaque valeur *C*₁, *C*₂, .... *C*ₙ,
(e₀) la détermination de la valeur β de *i,* telle que βₘᵢₙ < β < βₘₐₓ et βₘᵢₙ et βₘₐₓ correspondent aux valeurs de *i* au point d'inflexion de la courbe *i =* f *(t)* pour respectivement *C*ₙ et *C*₁,
(f₀) le tracé de la courbe de référence *t*_{β} = f (*C*) pour la valeur *i* = β.

Le procédé de l'invention est parfaitement adapté à l'analyse de nombreux échantillons en temps réel, car il présente les avantages suivants :
- il est simple,
- il est rapide,
- il est précis,
- il est standardisé et
- il est automatisable.

En effet, le procédé de l'invention ne nécessite pas la dilution des échantillons ; par conséquent, il est particulièrement adapté à l'analyse de nombreux échantillons comme une banque de vecteurs de transfert de gènes. Par exemple, alors que pour déterminer le titre de 30 échantillons de vecteurs, les techniques de l'art antérieur nécessitent, la réalisation de 10 à 20 dilutions et donc la manipulation de 300 à 600 échantillons de cellules à chaque étape de la technique (infection, lyse, fixation, coloration, addition de substrat, hybridation), ce qui implique 1800 à 2400 manipulations pour une technique comprenant 3 étapes (infection, lyse ou fixation et coloration ou addition de substrat), le procédé de l'invention ne nécessite pas de dilution de l'échantillon, ni de manipulation des cellules et implique simplement 30 mesures du signal *i*. Par conséquent, contrairement, aux techniques de l'art antérieur, le procédé de l'invention est simple, parfaitement standardisé et automatisable.

Le procédé de l'invention est plus précis que les techniques de l'art antérieur, car dans ledit procédé les mesures sont effectuées à partir d'un même échantillon, pris à différents instants t, alors que dans les techniques de l'art antérieur les différentes dilutions de l'échantillon sont testées de façon indépendante, ce qui introduit des variations internes entre ces différentes dilutions.

Le procédé de l'invention qui utilise le temps comme paramètre variable, contrairement aux techniques de l'art antérieur, qui utilisent la concentration C comme paramètre variable, permet de déterminer la concentration des agents biologiques en temps réel en mesurant le signal *i* par des techniques comme la fluorimétrie, la luminométrie ou la spectrométrie, ce qui présente de nombreux avantages.

En effet, dans le procédé de l'invention, les valeurs expérimentales sont disponibles immédiatement ce qui permet d'avoir une estimation rapide de la concentration des vecteurs pour suivre la cinétique de production de ces vecteurs ou bien pour analyser rapidement une banque de vecteurs de transfert de gènes. En revanche, les techniques de l'art antérieur ne permettent pas de telles estimations étant donné qu'aucun résultat intermédiaire, en cours de test n'est disponible ; seul le résultat final est disponible, une fois que l'ensemble des données correspondant aux différentes dilutions ont été obtenues à l'instant t puis analysées, ce qui correspond à un délai de plusieurs jours à plusieurs semaines, en fonction de la technique utilisée.

Selon encore un autre mode de mise en oeuvre avantageux dudit procédé, ledit signal est sélectionné dans le groupe constitué par la fluorescence, la luminescence, l'absorbance ou le dénombrement de cellules.

De manière non-limitative, le signal est avantageusement mesuré par une technique telle que : la microscopie optique ou de fluorescence, la fluorimétrie, la luminométrie et la spectrométrie.

La présente invention a également pour objet un kit ou une trousse de dosage (titrage) ou de détection d'un agent biologique, caractérisée en ce qu'elle comprend :
- des cellules cibles vivantes à une concentration D constante,
- un agent biologique de référence de concentration C connue, ledit agent biologique étant sélectionné dans le groupe constitué par les virus et les vecteurs viraux de transfert de gènes, et
- la courbe de référence dudit agent biologique *t* β = f (*C*).

Une telle trousse de dosage doit être associée, pour mesurer l'intensité du signal de la réaction agent biologique + cellules cibles vivantes, à un moyen physique approprié.

Cette trousse de dosage qui permet des mesures en temps réel, est particulièrement adaptée, notamment à la détermination du titre (titrage) d'un vecteur utilisé en thérapie génique, d'un virus utilisé pour la production d'un vaccin, d'une protéine recombinante utilisée pour la production d'un produit biologique (médicament, réactif) ou bien au dosage et/ou à la détection d'un virus, pour le diagnostic d'une infection virale.

Outre les dispositions qui précèdent, l'invention comprend encore d'autres dispositions, qui ressortiront de la description qui va suivre, qui se réfère à des exemples de mise en oeuvre du procédé objet de la présente invention ainsi qu'aux dessins annexés, dans lesquels :
- la figure 1 représente les valeurs expérimentales de l'intensité du signal de fluorescence *i,* en fonction du temps *t* pour chaque concentration (conc.) d'un vecteur rétroviral codant la protéine fluorescente (EGFP). Les concentrations sont exprimées pour 10⁶ particules infectieuses /ml.
- la figure 2 représente les courbes *i* = f (*t*), déterminées à partir des valeurs expérimentales présentées à la figure 1.
- la figure 3 représente les valeurs *t*_{β} pour les différentes concentrations de vecteur, déterminées à partir de la courbe de la figure 2, pour la valeur β = 100 de *i*.
- la figure 4 représente la courbe de référence du vecteur rétroviral *t*_{β} = f (*C*), déterminée à partir des valeurs expérimentales présentées à la figure 3.
- la figure 5 représente les valeurs expérimentales de l'intensité du signal d'hybridation *i*, en fonction du temps *t*, pour chaque dilution d'un vecteur associé à l'adénovirus recombinant (AAVr).
- la figure 6 représente les courbes *i* = f *(t),* déterminées à partir des valeurs expérimentales présentées à la figure 5.
- la figure 7 représente les valeurs *t*_{β} pour les différentes concentrations de vecteur, déterminées à partir de la courbe de la figure 6, pour la valeur β = -2 de *i*.
- la figure 8 représente la courbe de référence du vecteur AAVr, *t*_{β} = f (C), déterminée à partir des valeurs expérimentales présentées à la figure 7.

Il doit être bien entendu, toutefois, que ces exemples sont donnés uniquement à titre d'illustration de l'objet de l'invention, dont ils ne constituent en aucune manière une limitation.

### EXEMPLE 1 : Détermination du titre d'un vecteur rétroviral dans des cellules Rat-2.

### 1.1-Matériels et méthodes

L'agent biologique analysé est un vecteur rétroviral dénommé pSI-EGFP (Ropp et al., Cytometry, 1995, 21, 309-317), codant le gène rapporteur de la protéine fluorescente eucaryote *(Eukaryotic Green Fluorescent Protein* ou EGFP), les cellules cibles sont les cellules Rat-2 (ATCC CRL1764) et la réaction agent biologique + cellules cibles vivantes est l'expression du gène rapporteur EGFP. Le produit P qui est mesuré pour déterminer le titre dudit vecteur (concentration en particules rétrovirales infectieuses ou *ip*) est la quantité de protéine EGFP, qui est mesurée par fluorimétrie.

Des cellules Rat-2 sont ensemencées dans les puits d'une plaque de microtitration à une concentration constante, puis infectées à *t* = 0, avec les dilutions 1/2, 1/4 et 1/10 d'une préparation de vecteur rétroviral de référence de concentration initiale connue (C₀ = 10⁶ particules infectieuses /ml ), correspondant respectivement aux concentrations de 0,5.10⁶ ; 0,25.10⁶ et 0,1.10⁶ particules infectieuses/ml. Aux différents instants *t* = 16 h, 24 h, 40 h, 48 h et 64 h, l'intensité du signal de fluorescence *i*, émis par les cellules infectées par chaque dilution de vecteur est mesurée par fluorimétrie. La courbe *i* = f (*t*) est ensuite tracée pour chaque dilution et la valeur β est fixée à 100. Les valeurs *t*_{β}, correspondant aux valeurs de t lorsque *i* = 100, sont déterminées pour chaque concentration d'échantillon de référence, puis, la courbe t_{β} = f (C) est tracée à partir de ces valeurs.

### 1.2-Résultats

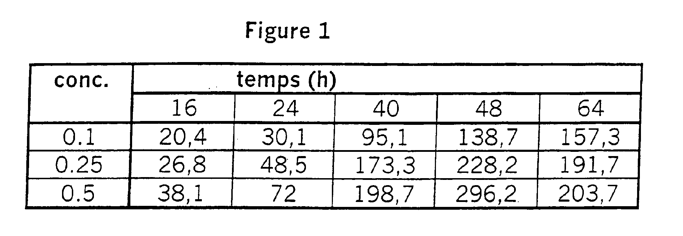

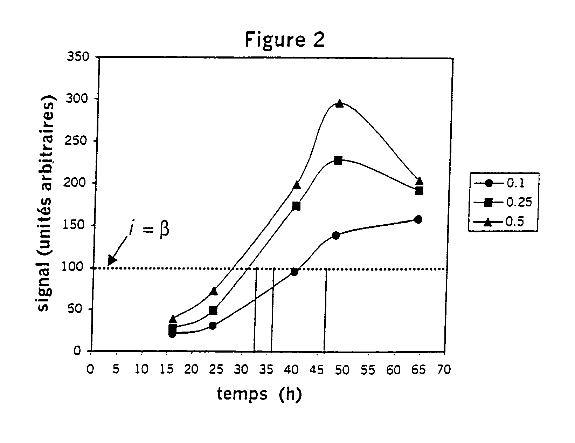

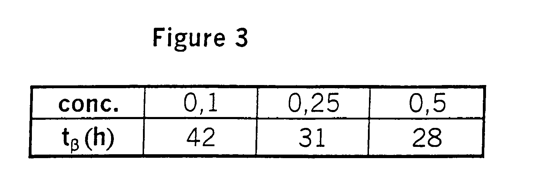

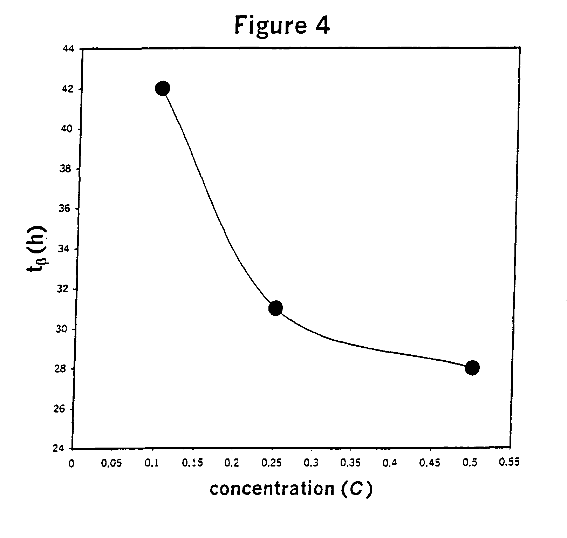

La figure 2 représente la courbe *i* = f (*t*), déterminée à partir des valeurs expérimentales présentées à la figure 1. La figure 4 représente la courbe de référence du vecteur rétroviral, *t*_{β} = f (*C*), déterminée à partir des valeurs expérimentales présentées à la figure 3.

La figure 2 montre que l'intensité du signal est une fonction du temps t d'incubation du vecteur avec les cellules cibles et de la concentration C dudit vecteur.

La figure 2 montre également que la valeur β=100, permet une détermination sensible et précise de la concentration du vecteur car elle détecte de faibles concentrations de vecteur et pour cette valeur, les variations de C correspondent à une variation importante de t. Ces résultats sont confirmés par la figure 4 qui représente la courbe de référence du vecteur, *t*_{β} = f (*C*). Cette courbe de référence montre qu'il existe une relation directe entre les valeur de *t*_{β} et de *C* qui permet de déterminer la concentration d'une préparation de vecteur, sans dilution et sans manipulation des cellules, simplement par détermination de la valeur expérimentale t_{β} de ce vecteur, correspondant à la valeur de β = 100.

### EXEMPLE 2: Comparaison du titre d'un vecteur associé à l'adénovirus recombinant (AAVr) déterminé à l'aide des paramètres d'un procédé classique (temps constant et concentration variable) ou du procédé de l'invention (temps variable et concentration constante).

### 2.1-Matériels et méthodes

L'agent biologique analysé est un virus associé à l'adénovirus, recombinant (AAVr), codant le gène rapporteur lacZ, les cellules cibles sont la lignée Hela-repcap32, la réaction agent biologique + cellules cible vivantes est la réplication virale et le produit P qui est mesuré pour déterminer le titre du vecteur (concentration en particules actives ou *ip*) est le nombre de copies de génome de AAVr. P est mesuré par hybridation de type Dot-blot selon les techniques classiques connues de l'Homme du métier.

Les cellules Hela-repcap32 sont ensemencées dans les puits d'une plaque de microtitration à une concentration constante, puis co-infectées, à *t* = 0, avec les dilutions 10⁻⁴, 10⁻⁷, 10⁻⁸ et 10⁻⁹ du vecteur et l'adénovirus sauvage, à une multiplicité d'infection de 100.

Aux différents instants *t* = 6 h, 14 h, 18 h, 20 h, 24 h, 30 h, 38 h, 44 h, 48 h et 54 h, les cellules sont récoltées puis le génome viral est isolé et hybridé avec une sonde nucléotidique spécifique marquée, selon la technique du Dot Blot classiquement utilisée par l'homme du métier. L'intensité du signal, qui représente la quantité d'ADN hybridée est mesurée à l'aide d'un phosphorimageur.

Pour la détermination du titre par le procédé classique, la courbe log *i* = f (log dilution) est tracée à partit des valeurs obtenues au temps *t =* 24 h et le titre est déterminé par approximation asymptotique dans la région des plus fortes dilutions.

Pour la détermination du titre par le procédé de l'invention, la courbe log *i* = f (*t*) est tracée pour chaque dilution de vecteur et la valeur β est fixée à - 2. Les valeurs *t*_{β}, correspondant aux valeurs de t lorsque *i* = -2, sont déterminées pour chaque concentration de l'agent de référence, puis, la courbe *t*_{β} = f (log C) est tracée à partir de ces valeurs.

### 2.2-Résultats

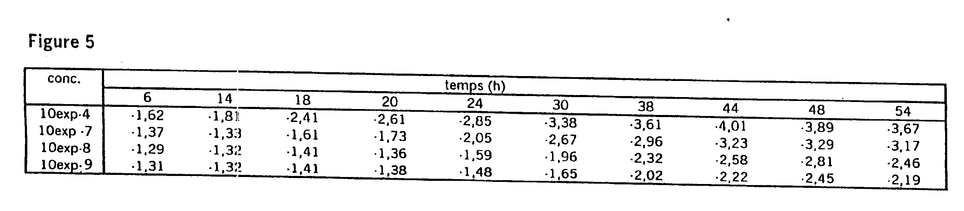

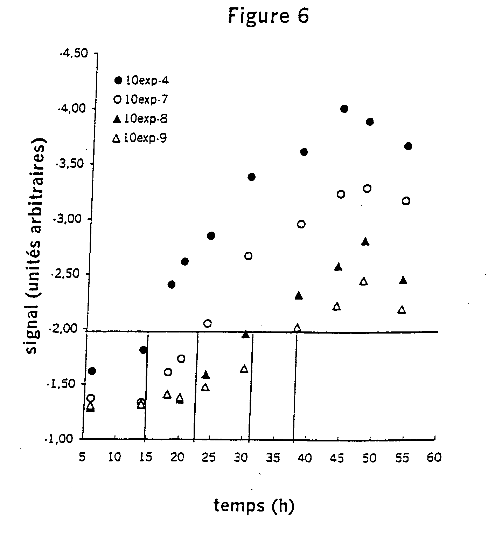

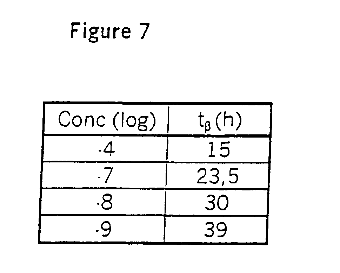

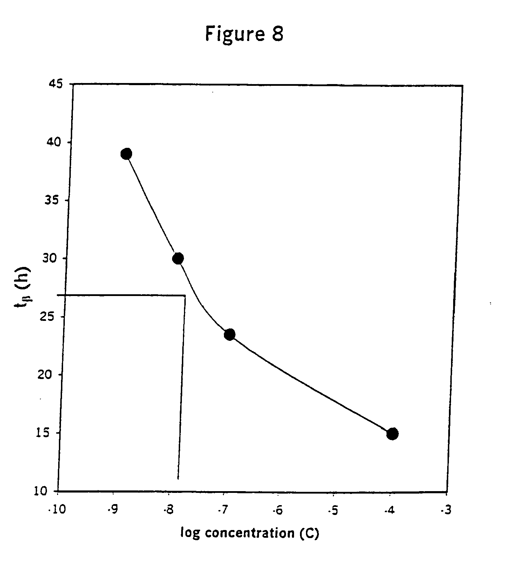

La figure 6 représente la courbe log *i* = f (*t*), déterminée à partir des valeurs expérimentales présentées à la figure 5. La figure 8 représente la courbe de référence du vecteur AA Vr, *t*_{β} = f (log *C*), déterminée à partir des valeurs expérimentales présentées à la figure 7.

La figure 6 montre que l'intensité du signal est une fonction du temps t d'incubation du vecteur avec les cellules cibles et de la concentration C dudit vecteur.

La figure 6 montre également que la valeur β= - 2, permet une détermination sensible et précise de la concentration du vecteur, car elle détecte de faibles concentrations de vecteur et pour cette valeur, les variations de C correspondent à une variation importante de t.

Ces résultats sont confirmés par la figure 8 qui représente la courbe de référence du vecteur, *t*_{β} = f (log*C*). Cette courbe de référence montre qu'il existe une relation directe entre les valeurs de *t*_{β} et de *C* qui permet de déterminer, avec précision et sans dilution, la concentration d'une préparation de vecteur, à partir d'une seule valeur expérimentale t_{β} de ce vecteur correspondant à la valeur de β = -2.

A titre comparatif, pour une même préparation de vecteur, le procédé classique donne un titre de 1 × 10⁸ particules infectieuses/ml et le procédé de l'invention donne un titre de 0,85 x 10⁸ particules infectieuses/ml. Ces résultats montrent que les valeurs obtenues par le procédé de l'invention sont comparables à celles obtenus par les procédés classiques de titrage d'agents biologiques.

Néanmoins, contrairement aux procédés classique le procédé de détermination du titre d'agent biologiques de l'invention permet avantageusement d'analyser de nombreux échantillons, simultanément et en temps réel. En effet, il est simple, rapide, précis, standardisé, automatisable et la valeur de l'intensité du signal qui mesure le produit de la réaction de l'agent biologique avec les cellules cibles vivantes est obtenue instantanément, sans intervention sur les cellules cibles et sur la réaction biologique dont le produit est mesuré.

Ainsi, le procédé de l'invention est utilisable aussi bien pour :
- cribler rapidement une banque de vecteurs de transfert de gènes ou de mutants d'une protéine recombinante,
- optimiser ou contrôler la production : des vecteurs de transfert de gènes thérapeutiques utilisables pour des essais précliniques et des essais cliniques de phase I, des virus utilisés comme vaccins viraux ou des protéines recombinantes utilisées comme médicament ou comme réactif biologique et
- détecter rapidement une infection virale à partir d'un échantillon biologique d'un patient à tester.

## Revendications

1. Procédé de détermination du titre d'un agent biologique interagissant avec des cellules cibles vivantes, sélectionné dans le groupe constitué par les virus et les vecteurs viraux de transfert de gènes, ledit procédé étant **caractérisé en ce qu'**il comprend au moins les étapes suivantes :
(a₁) l'incubation dudit agent biologique à une concentration C initiale inconnue, avec lesdites cellules cibles à une concentration D constante,
(b₁) la mesure à différents temps successifs t, de l'intensité *i* d'un même signal, qui résulte de la réaction agent biologique + cellules cibles vivantes,
(c₁) la détermination du temps *t*_{β} correspondant à la valeur *i* = β, choisie dans l'intervalle βₘᵢₙ < β < βₘₐₓ, tel que βₘᵢₙ et βₘₐₓ correspondent aux valeurs de *i* au point d'inflexion de la courbe *i* = f (*t*) pour respectivement les concentrations minimales et maximales d'un agent biologique de référence dont la courbe *t*_{β} = f (C) est préétablie et
(d₁) la détermination de la concentration C initiale de l'agent biologique, à l'aide de ladite courbe de référence t_{β} = f(*C*).

2. Procédé selon la revendication 1 **caractérisé en ce que** simultanément ou préalablement à l'étape (a₁), ladite courbe de référence est établie selon les étapes suivantes :
(a₀) la préparation, de n dilutions en série d'un agent biologique de référence de concentration *C₀* initiale connue, correspondant respectivement aux concentrations finales *C*₁, *C*₂,...... *C*ₙ dudit agent biologique,
(b₀) l'incubation de chaque dilution dudit agent biologique de référence obtenue en a₀ avec lesdites cellules cibles à une concentration D constante,
(c₀) la détermination à différents temps successifs *t*₁ à *t*ₙ de l'intensité du signal *i* qui résulte de la réaction agent biologique+ cellules cibles vivantes, pour chaque concentration *C*₁, *C*₂,..... *C*ₙ dudit agent biologique de référence,
(d₀) le tracé de la courbe *i* = f (*t*) pour chaque valeur *C*₁, *C*₂, .... *C*ₙ,
(e₀) la détermination de la valeur β de *i*, telle que βₘᵢₙ < β < βₘₐₓ et βₘᵢₙ et βₘₐₓ correspondent aux valeurs de *i* au point d'inflexion de la courbe *i* = f *(t)* pour respectivement Cₙ et C₁,
(f₀) le tracé de la courbe de référence *t*_{β} = f(*C*) pour la valeur *i* = β

3. Procédé selon la revendication 1 ou la revendication 2à, **caractérisé en ce que** ledit signal est sélectionné dans le groupe constitué par la fluorescence, la luminescence, l'absorbance et le dénombrement de cellules.

4. Trousse de dosage ou de détection d'un agent biologique, **caractérisée en ce qu'**elle comprend :
- des cellules cibles vivantes à une concentration D constante,
- un agent biologique de référence de concentration C connue, ledit agent biologique étant sélectionné dans le groupe constitué par les virus et les vecteurs viraux de transfert de gènes, et
- la courbe de référence dudit agent biologique *t* β = f (*C*).

## Claims

1. Method for determining the titer of a biological agent which interacts with living target cells, selected from the group consisting of viruses and viral gene transfer vectors, said method being **characterized in that** it comprises at least the following steps:
(a₁) the incubation of said biological agent at an unknown initial concentration C, with said target cells at a constant concentration D,
(b₁) the measurement at various successive times t of the intensity *i* of the same signal, which results from the reaction biological agent + living target cells,
(c₁) the determination of the time *t*_{ß} corresponding to the *i* = β value, chosen in the interval ßₘᵢₙ < ß < ßₘₐₓ, wherein ßₘᵢₙ and ßₘₐₓ respectively correspond to the values of *i* at the inflexion point of the *i* = f (*t*) curve for the minimal and maximal concentrations of a standard biological agent for which the *t*_{ß} = f (*C*) curve is preestablished, and
(d₁) the determination of the initial concentration *C* of the biological agent, using said *t*_{β} = f (*C*) standard curve.

2. Method according to claim 1, **characterized in that** simultaneously or before the step (a₁), said standard curve is established according to the following steps:
(a₀) the preparation of n serial dilutions of a standard biological agent at a known initial concentration C₀, corresponding to said biological agent final concentrations *C*₁, *C*₂, ... *C*ₙ, respectively,
(b₀) the incubation of each standard biological agent dilution obtained in (a₀) with said target cells at a constant concentration *D,*
(c₀) the determination of the intensity *i* of the signal which results from the reaction biological agent + living target cells, for each standard biological agent concentration *C*₁, *C*₂ , ... *C*ₙ at successive times *t*₁ to *t*ₙ,
(d₀) the drawing of the *i* = f (*t*) curve for each value of *C*₁, *C*₂, ... Cₙ,
(e₀) the determination of the β value of *i*, chosen in the interval βₘᵢₙ < β < βₘₐₓ, wherein βₘᵢₙ and βₘₐₓ respectively correspond to the values of *i* at the inflexion point of the *i* = f (*t)* curve for *C*ₙ and *C*₁,
(f₀) the drawing of the *t*_{β} = f (C) curve for the *i* = β value.

3. Method according to claim 1 or claim 2, **characterized in that** said signal is selected from the group consisting of fluorescence, luminescence, absorbance and cell numeration.

4. Kit for the quantification or the detection of a biological agent, **characterized in that** it comprises:
- living target cells at a constant concentration D,
- a standard biological agent of known concentration C, said biological agent being selected from the group consisting of viruses and viral gene transfer vectors, and
- the *t*_{β} = f (*C*) standard curve of said biological agent.

## Patentansprüche

1. Verfahren zum Bestimmen des Gehalts einer biologischen, mit lebenden Zielzellen interagierenden Substanz, ausgewählt aus der Gruppe, bestehend aus Viren und viralen Vektoren für den Gentransfer, wobei des Verfahren **dadurch gekennzeichnet ist, dass** es wenigstens die folgenden Schritte umfasst:
(a₁) Inkubieren der biologischen Substanz einer anfänglich bekannten Konzentration C, mit den Zielzellen einer konstanten Konzentration D,
(b₁) Messen der Intensität *i* eines gleichen Signals, das aus der Reaktion der biologischen Substanz mit den lebenden Zielzellen resultiert, zu verschiedenen aufeinander folgenden Zeiten t,
(c₁) Bestimmen der, die dem Wert *i* = β entsprechenden Zeit *t*_{β}, ausgewählt aus dem Intervall βₘᵢₙ < β < βₘₐₓ, so dass βₘᵢₙ und βₘₐₓ den Werten von *i* am Punkt der Biegung der Kurve *i* = f(t) für jeweils die minimalen und maximalen Konzentrationen einer biologischen Referenzsubstanz, deren Kurve *t*_{β} = f(*C*) vorbestimmt ist, entsprechen,
(d₁) Bestimmen der anfänglichen Konzentration C der biologischen Substanz mit Hilfe der Referenzkurve t_{β} = f(C),

2. Verfahren nach Anspruch 1, **dadurch gekennzeichnet, dass** gleichzeitig oder vor dem Schritt (a₁) die Referenzkurve nach den folgenden Schritten festgelegt wird:
(a₀) Herstellen von n Verdünnungsreihen einer biologischen Referenzsubstanz einer anfänglich bekannten Konzentration C₀, die jeweils den Endkonzentrationen *C₁, C₂,... Cₙ* der biologischen Substanz entspricht,
(b₀) Inkubieren einer jeden der in (a₀) mit den Zielzellen einer konstanten Konzentration D erhaltenen Verdünnung der biologischen Referenzsubstanz,
(c₀) Bestimmen der Intensität des Signals *i*, das aus der Reaktion der biologischen Substanz mit den lebenden Zielzellen resultiert, zu verschiedenen aufeinanderfolgenden Zeiten t₁ bis tₙ, für jede Konzentration *C₁, C₂,* ... Cₙ der biologischen Referenzsubstanz,
(d₀) Zeichnen der Kurve *i* = *f(t)* für jeden Wert *C₁, C₂,* ... *Cₙ,*
(e₀) Bestimmen des Werts β von *i*, so dass βₘᵢₙ < β < βₘₐₓ und βₘᵢₙ und βₘₐₓ den Werten von *i* am Punkt der Biegung der Kurve *i* = f(t) für jeweils *Cₙ* und *C₁* entsprechen,
(f₀) Zeichnen der Referenzkurve *t*_{β} = f(C) für den Wert *i* = β.

3. Verfahren nach Anspruch 1 oder 2, **dadurch gekennzeichnet, dass** das Signal ausgewählt ist aus der Gruppe, bestehend aus Fluoreszenz, Lumineszenz, Absorptionsvermögen und Zellzählung.

4. Untersuchungs-Kit und Sceeningverfahren einer biologischen Substanz, **dadurch gekennzeichnet, dass** es folgendes umfasst:
- lebende Zielzellen einer konstanten Konzentration D,
- eine biologische Referenzsubstanz einer bekannten Konzentration C,
wobei die biologische Substanz ausgewählt ist aus der Gruppe, bestehend aus Viren und viralen Vektoren für den Gentransfer, und
- eine Referenzkurve der biologischen Substanz *t*_{β} *=* f(*C*).
